# EUROPEAN PATENT APPLICATION

(11) **EP 2 975 026 A1**
(43) Date of publication of application: **20.01.2016**
(21) Application number: 14177421.6
(22) Date of filing: 17.07.2014
(51) Int. Cl.: C07D 213/76, C07C 201/00, C07C 209/68, C07D 239/52

(54) **Method of nitrosation of organic compound**

(71) Applicant: DSM Fine Chemicals Austria Nfg GmbH & Co KG, 4021 Linz (AT)
(72) Inventor: Hermsen, Petrus, Johannes, 6100 AA Echt (NL); Poechlauer, Peter, 6100 AA Echt (NL)
(74) Representative: Hansen, Jesper Römer

(57) **Abstract**

Method of nitrosation of an aromatic organic compound, involving continuous preparation of dinitrogen trioxide in a microreactor, and continuous reaction of dinitrogen trioxide with the aromatic organic compound.

## Description

The invention relates to a method of nitrosation of an organic compound. More particularly, the invention relates to nitrosation of an aromatic organic compound.

R02108864 discloses a process for the preparation of p-nitrosophenol from phenol, sodium nitrite and sulfuric acid. This process produces salts in addition to the desired nitrosophenol, which in the process according to the invention is considered a waste products.

RU 2129117 concerns a method of preparation of p-nitrosophenol from nitrogen trioxide and phenol. This patent discloses and claims the nitrosation of a suspension of phenol in water by N₂O₃ in the presence of a catalyst. RU 2127117 does not disclose continuous preparation of N₂O₃ and discloses that presence of ammonium chloride or dilute hydrochloric acid is required as catalyst.

RU2461543 discloses a method of manufacturing paracetamol via a process involving reduction of p-nitrosophenol in ethyl acetate in the presence of a Pd/C-containing catalyst by hydrogen at 2.0-4.0 atm and temperature 20-50°, followed by acylation of the intermediate p-aminophenol and the end product recovery.

FR2533559B1 discloses a process for the preparation of N-acetyl-p-aminophenol by simultaneous hydrogenation and acetylation of nitrosophenol in a solvent medium comprising at least one ester of an alcohol containing from 1 to 6 carbon atoms and/or at least one aliphatic organic acid containing from 1 to 5 carbon atoms.

It is an object of the invention to provide an improved method of nitrosation of an aromatic organic compound.

In another aspect of the invention, it is an object of the invention to provide a method of manufacturing N-(4-hydroxyphenyl)acetamide (APAP).

The improvement may for example be one or more of improved process mass intensity, improved safety, and/or reduced environmental impact.

One or more of the above and/or other objects of the invention are realised by a method according to claim 1.

### Brief description of the drawings

The invention will be explained more fully below with reference to exemplary embodiments as well as the figures, in which
Figure 1 shows an experimental set-up for the continuous nitrosation reaction, and
Figure 2 shows an alternative experimental set-up for the continuous nitrosation reaction.

The terms N-acetyl-p-aminophenol, N-(4-hydroxyphenyl)acetamide, paracetamol and APAP refers to the same chemical compound and are used interchangeably herein.

Process mass intensity (PMI) is herein meant as the total mass of materials used to produce a specified mass of product. Materials include reactants, reagents, catalysts and solvents used for reaction, isolation and purification *(Org.Proc.Res.Dev.* **2011,** *15,* 912).

The process according to the invention involves nitrosation of an aromatic compound. The nitrosation is conducted by reacting said compound with dinitrogen trioxide, N₂O₃, which N₂O₃ has been prepared in a continuous reaction between nitrogen monoxide, NO, and oxygen, O₂, where NO and O₂ are fed to a flow reactor at reduced temperature, such as -50°C to 0°C. Gaseous NO and O₂ are fed continuously to the reactor while N₂O₃ is removed. The use of flow reactor allows for a very low reactor inner volume and hence provides for a safe working environment where only small amounts of the toxic and air pollutant NO is present in the reactor at any time. The reaction between NO and O₂ may be conducted at ambient pressure, but it is preferred to conduct the reaction at between 2 to 10 bar to speed up the reaction. During the reaction between NO and O₂, additional (inert) gasses may be present, such as N₂.

By reacting nitrogen monoxide with oxygen in a continuous process is herein meant that the reaction in which new materials (NO and O₂) are added and product (N₂O₃) removed continuously at a rate that maintains the reaction volume at a specific level. In other words, continuous reactors that may be used carry out steady state operations.

By solvent for the aromatic organic compound is herein meant a solvent having a solubility of at least 10 g of the aromatic organic compound to be nitrosated by the method according to the invention per 1000 g solvent at 25°C and 1 bar. It was found to be advantageous that the solvent is selected from the group consisting of water, ethanol, methanol, acetic acid and a mixture thereof. Hence, preferred that the aromatic organic compound has a solubility of at least 10 g of the aromatic organic compound per 1000 g solvent at 25°C and 1 bar in at least one of the solvents selected from the group consisting of water, ethanol, methanol, acetic acid and a mixture thereof.

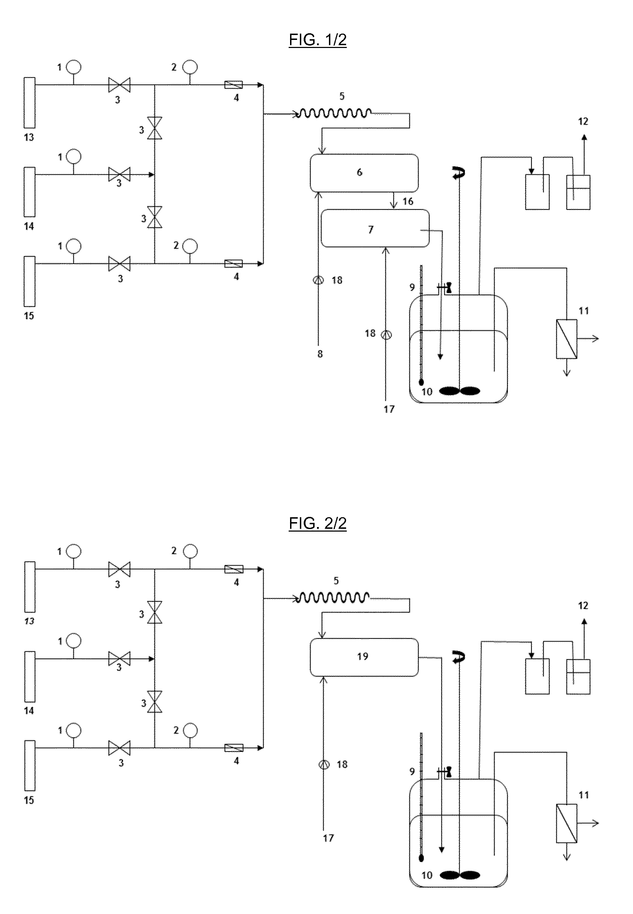

In Figure 1, an example of an experimental setup suitable for conducting the process according the invention where N₂O₃ is generated continuous and thereafter used for nitrosation of the aromatic organic compound is shown. The raw materials, oxygen gas, 13, and nitric oxide, 15, are supplied via individual pressure indicator and controllers, 1, and two-way valves, 3. The mass flow to the tubular flow reactor, 5, is determined by indicator and controller, 2, and enters the tubular flow reactor, 5, via back pressure valve 4, which prevent back flow of reacted reactants.

In the tubular flow reactor, 5, oxygen and nitric oxide reacts to form N₂O₃ in liquid state, which N₂O₃ is continuously flowed to a first plate reactor, 6, (such as a Corning plate reactor). In the first plate reactor, 6, the N₂O₃ reacts with water, 8, introduced to the plate reactor by a pump, 18, to form an aqueous solution of nitrous acid, 16. The aqueous solution of nitrous acid, 16, is transported to a second plate reactor, 7, where the aqueous solution of nitrous acid, 16, reacts with the substrate (aromatic organic compound), 17, introduced to the plate reactor, 7, by a pump, 18, to form an nitrosated organic compound. The nitrosated organic compound is transferred to a stirred batch reactor, 10 used to precipitate the nitrosated organic compound, which later is separated from the reaction fluid by filtration in a filtration unit, 11. The conditions of the batch reactor, 10, is monitored by thermometer, 9.

Unreacted gas, 12, is exhausted from the batch reactor leaves scrubbers containing aqueous base such as NaOH. After completion of the nitrosation of the phenol, the reaction product (nitrosophenol) is extracted from the reaction mixture by filtration and optionally purified by recrystallization. Furthermore, the tubular flow reactor, 5, may be purged for example by nitrogen gas, 14, via dedicated two-way valve, 3, pressure indicator and controller, 1.

In Figure 2, an example of an experimental setup suitable for conducting the process according the invention where N₂O₃ is generated continuous and thereafter used for nitrosation of the aromatic organic compound is shown. The raw materials, oxygen gas, 13, and nitric oxide, 15, are supplied via individual pressure indicator and controllers, 1, and two-way valves, 3. The mass flow to the tubular flow reactor, 5, is determined by indicator and controller, 2, and enters the tubular flow reactor, 5, via back pressure valve 4, which prevent back flow of reacted reactants.

In the tubular flow reactor, 5, oxygen and nitric oxide reacts to form N₂O₃ in liquid state, which N₂O₃ is continuously flowed to a plate reactor, 19, (such as a Corning plate reactor). In the plate reactor, 19, the N₂O₃ reacts with the substrate (aromatic organic compound), 17, introduced to the plate reactor, 19, by a pump, 18, to form an nitrosated organic compound. The nitrosated organic compound is transferred to a stirred batch reactor, 10 used to precipitate the nitrosated organic compound, which later is separated from the reaction fluid by filtration in a filtration unit, 11. The conditions of the batch reactor, 10, is monitored by thermometer, 9.

Unreacted gas, 12, is exhausted from the batch reactor leaves scrubbers containing aqueous base such as NaOH. After completion of the nitrosation of the phenol, the reaction product (nitrosophenol) is extracted from the reaction mixture by filtration and optionally purified by recrystallization. Furthermore, the tubular flow reactor, 5, may be purged for example by nitrogen gas, 14, via dedicated two-way valve, 3, pressure indicator and controller, 1.

A microreactor is generally defined as a miniaturized reactor with characteristic dimensions (channel of plate width) in micrometers to millimeters (preferably from 0.01 mm to 25.0 mm and more preferably from 0.1 mm to 20.0 mm). A number of microreactors may be combined in parallel to form a micro-structured reactor. Thus, the inner volume available for reaction depends on the diameter and length of the microreactor, or in case of a micro-structured reactor is used on the dimensions of the parallel channels and the number of parallel channels. The inner volume of microreactors or micro-structured reactors typically lies in the range of 1 ml to 1 m³, where 1m³ correspond to a very large number of parallel channels in a micro-structured reactor. Preferably, the inner volume of the microreactors or micro-structured reactors are from 10 ml to 50 l. Preferably, the inner volume of each microreactor is in the range of 1 ml to 200 ml and more preferably the inner volume of each microreactor is in the range of 2 ml to 100 ml.

It is preferred that at least one of the microreactors utilized in the method according to the invention is a flow microreactor. More preferably, the flow reactor is a plate flow microreactor or a tubular flow reactor, and most preferably the flow reactor is a tubular flow reactor.

Preferably, the microreactor has a channel with a hydraulic diameter of 20 mm or less. The hydraulic diameter Dh is defined as 4 A/U, wherein A is the inner cross sectional area of the reactor channel and U is the perimeter of said cross section. For a round tube, the hydraulic diameter equals the inner diameter of the tube. For a rectangular duct, that has a cross section with a rectangular shape, the hydraulic diameter equals 4 LW/2(L+W), wherein L is the length of the longest side of the rectangle and W is the width of the rectangle. For the special case of a square duct the hydraulic diameter equals L. It should be noted that the general formula 4 A/U allows the calculation of the hydraulic diameter for any shape of reactor channel.

A continuous microreactor is defined as a microreactor suitable for use in a continuous process. A continuous process is defined as a method of manufacturing in which new materials are added and products removed continuously at a rate that maintains the reaction volume at a specific level. In other words, continues reactors that may be used to carry out steady state operations.

The reaction between N₂O₃ and the aromatic organic compound may be conducted in various hydrophilic solvents like methanol, ethanol, acetic acid and water (or mixtures thereof), but it was found to be advantages to use an aqueous solution as it was surprisingly found that for aqueous solutions it was possible to conduct the reaction without the presence of a catalyst, which allows for an environmentally and economical advantage as compared to previous processes for nitrosation of aromatics. By catalyst is herein understood a compound added to enhance reaction speed without being consumed by the reaction.

After the reaction between N₂O₃ and the organic compound, the resulting nitrosated product is preferably separated from the reaction mixture by crystallization and filtration. It is preferred to conduct this separation at reduced temperature, such as at a temperature below 10°C and preferably at a temperature between -10 to 5°C. It was found that a yield of 4-nitrosophenol preferably should be at least 65 mol% based on the amount of aromatic organic compound. More preferably the yield of 4-nitrosophenol should be at least 75 mol%. Since the reaction between N₂O₃ and phenol will lead to some side reactions, it is preferred that the yield of the filtration is kept below 95 mol% and more preferably the yield is kept below 90 mol%.

Surprisingly it was found that using the method of the invention to manufacture nitrosated aromatics without catalyst allowed for low process mass intensity. Particularly it was found to be advantageous to conduct the method of manufacturing 4-nitrosophenol starting from phenol, nitrogen monoxide, oxygen and using water as solvent with a process mass intensity of less than 25, more preferably with a process mass intensity of less than 15, even more preferably with a process mass intensity of less than 10. In other words, the process according to the invention therefore represents a major advantage due to the very low environmental impact of the process as compared to methods of manufacturing 4-nitrosophenol till now.

Another aspect of the invention concerns a method of manufacturing aminophenol by hydrogenating 4-nitrosophenol obtained according to a method of the first aspect of the invention, i.e. by a process involving continuous reacting of nitrogen monoxide with oxygen to form dinitrogen trioxide and continuous nitrosating the aromatic organic compound by reacting the aromatic organic compound with the as prepared dinitrogen trioxide. It was found to be advantageous that the step of hydrogenating is conducted by reacting 4-nitrosophenol dissolved in a C1-C5 alcohol with H₂ gas in the presence of a catalyst. Preferably the C1-C5 alcohol contains at least 50 wt% methanol, such as 75 wt% or 90 wt%, and more preferably the C1-C5 alcohol is methanol. By C1-C5 alcohol is herein meant an alcohol with 1 to 5 carbon atoms, including methanol, ethanol, propanol, butanol, pentanol, Ethane-1,2-diol, Propane-1,2-diol, Propane-1,2,3-triol, Butane-1,2,3,4-tetraol, Pentane-1,2,3,4,5-pentol, isomers thereof and mixtures thereof.

The catalyst used in the hydrogenation may be any commonly used hydrogenation catalyst, however it is preferred to use a Pd/C catalyst, as this catalyst was found to be very active for this reaction.

A further aspect of the invention concerns method of manufacturing APAP by acylating aminophenol obtained by the method of the second aspect of the invention. Preferably acylation is realized by reacting aminophenol dissolved in a C1-C5 alcohol with acetic anhydride, Ac₂O. Preferably the C1-C5 alcohol contains at least 50 wt% methanol, such as 75 wt% or 90 wt%, and more preferably the C1-C5 alcohol is methanol. Alternative acylation agents that can be used, such as for example (but not limited to) acetyl chloride, acetyl bromide, methyl acetate, vinyl acetate and acetic acid, however, these may require presence of a catalyst during the process.

In a highly preferred embodiment, the hydrogenating of 4-nitrosophenol and acylating of aminophenol are conducted in a one pot reaction. By a one pot reaction is herein meant that the reaction is conducted in one reactor and at the same time.

Surprisingly it was found that using the method according to this aspect of the invention allowed for very low process mass intensity. Particularly it was found to be advantageous to conduct the method of manufacturing APAP starting from phenol, nitrogen monoxide, oxygen and acetic anhydride and using water and methanol as solvents (which solvents were recycled) with a process mass intensity of less than 40, more preferably with a process mass intensity of less than 30, even more preferably with a process mass intensity of less than 25. In other words, the process according to the invention therefore represents a major advantage due to the very low environmental impact of the process as compared to methods of manufacturing APAP till now.

It was found that the advantage of manufacturing the nitrosated organic compound by a reaction involving both continuous manufacturing of N₂O₃ and continuous nitrosation of the organic compound using the as prepared N₂O₃ can be achieved for a range of organic compounds. Preferably, the organic aromatic compound is selected from the group consisting of phenol derivatives, such as phenol, anisol and naphtol; benzoic acid derivatives, such as benzoic acid, methyl benzoate and salicylic acid; cinnamic acid derivatives, such as cinnamic acid and methyl cinnamate; benzene derivatives, such as benzene, toluene xylene and *N,N*-dimethyl benzene; pyridine derivatives, such as 2,6-diamino pyridine; benzaldehyde derivatives, such as benzaldehyde and 2-methylbenzaldehyde; pyrimidine derivatives, such as 2,6-dimethoxypyrimidin-4-amine; benzophenone derivatives, such benzophenone and 2-methoxybenzophenone; benzothiadiazole derivatives, such as benzothiadiazole and 2-methoxy benzothiadiazole; benzazepine derivatives, such as benzazepine and 9-methyl-1 H-benzo[b]azepine; 1,2,3,4-tetrahydroisoquinoline-1,3-dione derivatives, such as 1,2,3,4-tetrahydroisoquinoline-1,3-dione and 8-hydroxyisoquinoline-1,3-(2H,4H)-dione; quinoline derivatives, such as 8-methylquinoline adnd 8-hydroxyquinoline; indoline derivatives, such as 5,7-dimethylindoline and 7-methoxyindoline; phenylalanine derivatives, such as *N*-Boc-L-phenylalanine and N-acetyl metatyrosine; tetracycline derivatives, such as tetracycline, doxycycline and chlorotetracycline.

Particularly preferred organic compounds are the above mentioned phenol derivatives and most preferably the organic compound is phenol, since in this case the method can be used for the preparation of APAP, which is an important drug.

### EXAMPLES

### Example 1 - Preparation 4-nitrosophenol

In the experimental set-up depicted in Figure 1, NO gas (240 Nml/min) and O₂ gas (30 Nml/min) are dosed via a Bronkhorst dosing controller and mixed via a T-piece into a 2.5mtr. PTFE tube (ID=1.56mm, 4.7ml) under atmospheric pressure and ambient temperature. The gas mixture and water (5 ml/min) are dosed into a Corning glass reactor at 0°C. The nitrous acid solution leaving the first flow reactor and a 0.5 M aqueous solution of phenol (2.5 ml/min) are dosed to a second Corning glass flow reactor at 0°C. The outlet is collected in a 100ml double walled glass reactor at 2°C. Unconverted NOx gasses are removed via a basic scrubber. The precipitated 4-nitrosophenol is continuously isolated by filtration via a glass sintered filter. Thus, 4-nitrosophenol was obtained in 70 mol% isolated yield (relative to phenol).

### Example 2 - preparation of 2-hydroxy-5-nitrosobenzoic acid

In the experimental set-up depicted in Figure 2, NO gas (20 Nml/min) and O₂ (5 Nml/min) gas are dosed via a Bronkhorst dosing controller and mixed via a T-piece into a 2.5mtr. PTFE tube (ID=1.56mm, 4.7ml) under atmospheric pressure and ambient temperature. The gas mixture and water (5 ml/min) are dosed into a Corning glass reactor at 0°C. The nitrous acid solution leaving the first flow reactor and a 3.6 M solution of salicylic acid in water/acetic acid (1:4) (2.5 ml/min) are dosed to a second Corning glass flow reactor at 0°C. The outlet is collected in a 100ml double walled glass reactor at 2°C. Unconverted NOx gasses are removed via a basic scrubber. The precipitated 2-hydroxy-5-nitrosobenzoic acid is continuously isolated by filtration via a glass sintered filter. Thus, precipitated 2-hydroxy-5-nitrosobenzoic acid was obtained in 40 mol% isolated yield (relative to salicylic acid).

### Example 3 - Synthesis of N-acetyl 4-amino phenol (APAP)

In an autoclave, 4-NO-phenol (87.8 g) obtained in experiment 1 was dissolved in 878 mL methanol (11 wt% solution). To this solution, Ac₂O (2.0 equiv) and 4.39 grams Pd/C (5 wt%) were added. This mixture was brought under H₂ atmosphere (20 bar) and stirred for 1 hour at 50 °C showing complete conversion toward desired APAP. The crude reaction mixture obtained, consisted of >98.5% APAP and trace amounts of 4-aminophenol and over-acylated APAP.

The catalyst was removed by filtration and subsequently 100 mL of water was added to the filtrate. Methanol was removed *in vacuo* and the resulting mixture was cooled on ice. Crystallization of APAP was initiated by seeding with authentic APAP. The desired product was isolated as dark brown crystals upon filtration and washing with cold water. After drying 88 g (81.6 mol%) of APAP was obtained.

### Example 4

As shown in example 2 above, other substrates that phenol are also feasible in the process according to the invention. In Table 1, a number of preferred substrates, suggested solvents and conditions and resulting compounds are indicated.

**Table 1: Preferred embodiments.**

| **Substrate** | **Solvent** | **Temperature [°C]** | **Resulting compound** |
|---|---|---|---|
| 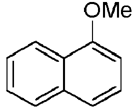 | Water/acetic acid | 20 | 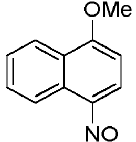 |
| 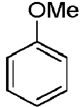 | Water | 20 | 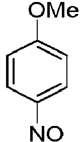 |
| 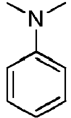 | Water | 20 | 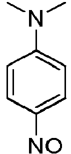 |
| 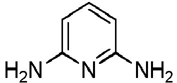 | Water | 20 | 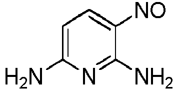 |
| 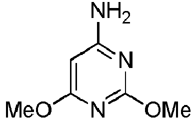 | Water | 20 | 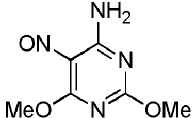 |

## Claims

1. A method of nitrosating an aromatic organic compound, comprising the steps of
- reacting nitrogen monoxide, NO, with oxygen, O₂, to form dinitrogen trioxide, N₂O₃,
- reacting dinitrogen trioxide with an aromatic organic compound in a solvent for the aromatic organic compound to obtain a nitrosated organic compound, wherein reacting nitrogen monoxide with oxygen is a continuous process and is conducted in a microreactor at ambient temperature to form liquid dinitrogen trioxide, and reacting dinitrogen trioxide with the aromatic organic compound is a continuous process.

2. Method according to claim 1, wherein the step of reacting dinitrogen trioxide with the aromatic organic compound is conducted in solution without presence of a catalyst.

3. Method according to any one of claims 1 or 2, further comprising the step of continuously separating the nitrosated organic compound from the solvent for the aromatic organic compound by a solid liquid separator, preferably the step of separating the nitrosated organic compound from the solvent for the aromatic organic compound has a yield of at least 65 mol% based on the aromatic organic compound.

4. Method according to any one of the claims 1 to 3, wherein the organic aromatic compound is selected from the group consisting of phenol derivatives, benzoic acid derivatives, cinnamic acid derivatives, benzene derivatives, pyridine derivatives, benzaldehyde derivatives, pyrimidine derivatives, benzophenone derivatives, benzothiadiazole derivatives, benzazepine derivatives, 1,2,3,4-tetrahydroisoquinoline-1,3-dione derivatives, quinoline derivatives, indoline derivatives, phenylalanine derivatives, tetracycline derivatives.

5. Method according to any one of the claims 1 to 4, wherein the aromatic organic compound has a solubility of at least 10 g of the aromatic organic compound per 1000 g solvent at 25°C and 1 bar in at least one of the solvents selected from the group consisting of water, ethanol, methanol, acetic acid and a mixture thereof.

6. Method according to any one of the claims 1 to 5, wherein the organic aromatic compound is a phenol derivate, preferably phenol.

7. Method according to any one of the claims 1 to 6, where the organic aromatic compound is phenol and further comprising the step of hydrogenating the 4-NO-phenol to obtain aminophenol, preferably by reacting 4-NO-phenol dissolved in an alcohol with H₂ gas in the presence of a Pd/C catalyst, and more preferably the alcohol is methanol.

8. Method according to claim 7 further comprising the step of acylating the aminophenol to obtain APAP, preferably by reacting aminophenol dissolved in an alcohol with acetic anhydride, Ac₂O, more preferably the alcohol is methanol.

9. Method according to claim 8, wherein hydrogenating the 4-NO-phenol and acylating the aminophenol is conducted in one batch reaction.

10. Method according to claim 8, wherein hydrogenating the 4-NO-phenol and acylating the aminophenol is conducted in a continuous process.

11. Method of manufacturing APAP by a method according to any one of the claims 1 to 10 from phenol, nitrogen monoxide, oxygen and acetic anhydride using water and/or methanol as solvents with a process mass intensity of less than 40, more preferably with a process mass intensity of less than 30, even more preferably with a process mass intensity of less than 25 under recycling of methanol.

12. Method according to any one of the claims 1 to 11, wherein the microreactor is a flow reactor, preferably the flow reactor has an inner width of between 0.1 mm to 20.0 mm and/or an inner volume of at least 10 ml.

13. Method according to claim 12, wherein the flow reactor is a tubular flow reactor.
